# EUROPEAN PATENT APPLICATION

(11) **EP 3 842 532 A1**
(43) Date of publication of application: **30.06.2021**
(21) Application number: 19219326.6
(22) Date of filing: 23.12.2019
(51) Int. Cl.: C12N 15/10, C12Q 1/6855

(54) **METHOD OF AMPLIFYING MRNAS AND FOR PREPARING FULL LENGTH MRNA LIBRARIES**

(71) Applicant: baseclick GmbH, 82061 Neuried (DE)
(72) Inventor: Frischmuth, Thomas, 12247 Berlin (DE); Serdjukow, Sascha, 83088 Kiefersfelden (DE); Sobotta, Jessica, 80336 München (DE); Graf, Birgit, 80469 München (DE)
(74) Representative: Weickmann & Weickmann PartmbB

(57) **Abstract**

The inventive method for amplifying at least one RNA which is contained in a sample includes reverse transcribing the at least one RNA using a first primer, adding a dideoxy nucleotide which is modified in the 3'-position with a first partner of a pair of azide and alkyne molecules by action of a template-independent polymerase to attach a single 3'-azide- or 3'-alkyne-modified dideoxy nucleotide at the 3'-end of the obtained cDNA, adding an adapter molecule which comprises a polynucleotide sequence and at its 5'-end a second partner of such pair of azide and alkyne molecules and ligating the adapter to the 3'-modified cDNA under formation of a triazole linkage, adding a second primer which is complementary to at least a part of the adapter molecule and which contains at its 3'-end a nucleotide which is complementary to the dideoxy nucleotide at the 3'-end of the cDNA to effect hybridization and binding of the second primer overlapping the triazole linkage, adding a third primer and amplifying the full length cDNA.

Uses of such method especially for preparing a full length RNA library and for sequencing of a plurality of RNAs contained in a sample, as well as reagent kits for perfoming such methods are also disclosed and included in the invention..

## Description

The present invention relates to methods for amplifying at least one mRNA contained in a sample, to the use of such method for preparing a full-length mRNA library from a sample containing a plurality of mRNA molecules, a method for sequencing the total mRNA of specific cells or the whole exome of an organism and to methods for genetic diagnosis. Further, the invention relates to a kit containing reagents for performing at least one of the methods of the invention.

### Background of the invention

Despite all technological advancements in recent years, RNA sequencing, especially full-length mRNA sequencing or transcriptome or exome sequencing have remained a tremendous challenge. The exome includes all parts of the genome of an organism which are contained in the exons and which, after transcription of the genetic information and removal of introns via RNA splicing, result in mRNA which is translated into the final proteins of such organism. The transcriptome, on the other hand, includes all RNAs of one cell or a specific population of cells, especially all corresponding mRNAs.

Whole exome sequencing can tremendously contribute to the understanding of common as well as rare diseases. Mutations which affect or even eliminate the function of specific proteins are known to be the major cause of Mendelian diseases. E.g. Choi et al. (Proc Natl Acad Sci USA, 106 (45), p. 19096-19101 (2009)) and Bamshad et al. (Nat Rev. Genet., 12, 745-755 (2011)) reported on whole exome capture and massively parallel DNA sequencing for determining genetic variations which play a role in Mendelian and non-Mendelian diseases.

The transcriptome of especially cancer cells is of particular importance for a better understanding of carcinogenesis. However, also the analysis of molecular mechanisms and cellular pathways in other cell types like stem cells is a permanent focus of research, as is biomarker discovery for a variety of different applications and uses.

While the human and animal exomes and transcriptomes are of interest especially to advance treatment or prevention of diseases, for plant cells additional interest lies in a better understanding of plant genetics e.g. for improvement of crop plants by targeted mutation and genetic engineering.

The rapid analysis of genetic material requires easy to use, efficient and reliable tools. A major problem is the need to detect DNA or RNA of interest directly in small biological samples such as patient blood or other sources. These provide the various components of the genetic material only in minute amounts. In order to reach the required sensitivity, an amplification step is usually required in which the amount of the nucleic acids in a sample is increased prior to analysis. Alternatively, a detection method is applied in which the minute detection signal which is directly obtained from the DNA/RNA analyte is amplified.

Methods for the amplification of nucleic acids include PCR amplification and other nucleic acid amplification protocols. PCR amplification has the major advantage that within the pool of different DNA strands obtained from a biological material only the DNA sequence of interest is amplified. This is the basis for a reliable analysis of single genes in complex biological samples. In the last few years, the focus has shifted to next generation sequencing (NGS) techniques and RNA sequencing. Since there is no robust method to sequence RNA directly, all library preparation methods start by reverse transcription of the mRNA into DNA. The resulting single-stranded cDNA is then usually converted into a double-stranded DNA (dsDNA) during the so-called "second-strand synthesis", which renders the oligonucleotides compatible with the sequencing machines. Moreover, adapter sequences are needed for the sequencing procedure. These adapters can only be introduced efficiently into dsDNA by tagmentation (transposase-mediated DNA fragmentation and primer addition) or via enzymatic ligation. This conversion process of single-stranded RNA into double-stranded DNA is the reason for the complexity of current RNA library preparation methods.

Recently, WO 2019/063803 disclosed a chemical ligation method which enables to chemically ligate single-stranded DNA with a similar efficiency as ligases show for dsDNA. The method uses the click chemistry concept which was independently defined by the groups of Sharpless and Meldal in 2001/2002 (Sharpless, K. B. et al., Angew. Chem. 2002, 114, 2708, Angew. Chem. Int. Ed. 2002, 41, 2596; Meldal, M. et al., J. Org. Chem. 2002, 67, 3057). The copper-catalyzed reaction of azides with alkynes to give 1,2,3-traizoles, which is a variation of the 1,3-dipolar Huisgen cycloaddition (R. Huisgen, 1,3,-Diploar Cycloaddition Chemistry (Ed.: A Padwa), Wiley, New York, 1984), has become the most widely used method to perform a click reaction. As a result of its mild conditions and high efficiency, this reaction has found a myriad of applications in biology and material sciences, such as e.g DNA labeling for various purposes (Gramlich, P.M.A. et al., Angew. Chem. Int. Ed. 2008, 47, 8350).

This chemical ligation principle has promoted various groups to develop library preparation methods without enzymatic ligation (Routh, A. et al., J. Mol. Biol. 427, 2610-2616 (2015), US 2018/0127816 A1 (Illumina, Inc.), Miura, F. et al., Nucleic Acids Research, Vol. 46, No. 16, e95 (2018*)).* Despite benefits in decreasing the number of protocol steps, e.g. by eliminating the need for a second strand synthesis or suppressing artefactual recombination and primer dimer formation, a slight decrease in sensitivity compared to conventional protocols was noted. Besides, due to the need to incorporate an artificial nucleotide during reverse transcription and as the resulting backbone mimic is not accepted by all polymerases employed in standard library preparation methods, broad commercial application of the new protocol is missing.

Accordingly, it was an object of the present invention to provide an improved concept for amplification of mRNAs and for preparation of full-length libraries especially of a plurality of mRNAs contained in a sample, e.g. a whole exome preparation or the total mRNA of a certain cell type of an individual. It was a further object to provide a method which exploits the efficiency of the chemical ligation method for joining of single-stranded DNA, but which is compatible with standard enzymes in library workflows.

### Summary of the invention

The present invention solves the above mentioned problem by providing an improved method for amplifying RNA, preferably mRNA, especially for preparation of a full length mRNA library for the total mRNA of certain cells or for the whole exome of an organism.

In a first aspect the invention relates to a method of amplifying at least one RNA, preferably mRNA contained in a sample, the method comprising:
a) providing at least one first primer, which first primer includes a sequence which is complementary to a sequence which is located at or near the 3'-end of the at least one mRNA to be amplified, and adding said at least one first primer to the sample under conditions which allow for hybridization of at least one first primer to at least one mRNA,
b) reverse transcribing the at least one mRNA under conditions including addition of a reverse transcriptase and nucleotides to the sample to provide full length cDNA(s) of the at least one mRNA,
c) purifying the sample at least from excess nucleotides
d) adding a dideoxy nucleotide, which dideoxy nucleotide is modified at the 3'-position to include a first partner of a pair of azide and alkyne molecules, and a template-independent polymerase to attach a single 3'-azide- or 3'-alkyne modified dideoxy nucleotide at the 3'-end of the cDNAs obtained in step b),
e) purifying the sample at least from excess modified dideoxy nucleotide added in step d),
f) adding an adapter molecule, the adapter molecule comprising a polynucleotide sequence and, attached to its 5' end, a second partner of said pair of azide and alkyne molecules, under conditions to perform a click reaction and to ligate the adapter molecule to the 3'-modified cDNAs obtained in step d) under formation of a triazole linkage,
g)) adding a second primer, the second primer comprising a nucleotide sequence which is complementary to at least 6 of the nucleotides at the 5'-end of the adapter molecule and contains at its 3'-end a nucleotide which is complementary to the dideoxy nucleotide at the 3'-end of the cDNAs, to effect hybridization and binding of the second primer to the ligated adapter/cDNA molecule obtained in step e) at a position overlapping the triazole linkage,
h) adding a third primer which is identical to at least a part of the first primer, and
i) amplifying the full length cDNAs.

In a second aspect, the invention relates to the use of such inventive method for preparing a full-length mRNA library from a sample containing a plurality of mRNA molecules.

In a third aspect the invention is directed to a method for sequencing the total mRNA of one or more types of cells of an organism or the whole exome of an organism, the method comprising providing a sample containing such exome or total cell mRNA, preparing a library of full-length mRNA by performing the method according to the invention as described herein and determining the sequence of the obtained total cell mRNA or exome.

In a fourth aspect, the invention relates to a kit for amplifying at least one RNA, preferably mRNA contained in a sample, the kit comprising:
a) a first primer which primer includes a sequence which is complementary to a sequence which is located at or near the 3'-end of the at least one mRNA to be amplified,
b) a reverse transcriptase,
c) a dideoxy nucleotide which is modified at the 3' position to include a first partner of a pair of azide and alkyne molecules,
d) a template-independent polymerase,
e) an adapter molecule comprising a polynucleotide sequence and attached to its 5'-end a second partner of said pair of azide and alkyne molecules
f) a second primer comprising a nucleotide sequence which is complementary to at least 6 of the nucleotides at the 5'-part of the adapter molecule and which contains at its 3'-end a nucleotide which is complementary to the dideoxy nucleotide of c),
g) a third primer which is identical to at least a part of the first primer.

### Detailed description of the invention and of preferred embodiments

Theamplification of full-length mRNA from natural sources, especially the total mRNA of specific cells or cell types or even the whole exome of anorganism, i.e. the complete coding regions of protein encoding genes, via commonly used methods is still a complicated and time-consuming procedure. The present invention employs so-called "click chemistry" to facilitate amplification of a full-length mRNA, and even the amplification of several or a multitude of mRNAs contained in a sample can be performed in one single procedure.

Shortly summarized, the invention includes cDNA preparation from the one or more mRNA templates via reverse transcription, during or after which the obtained one or more cDNAs are modified at their 3'-end to include an alkyne- or an azide-modification. More specifically, one partner of a pair of molecules which participate in a click reaction is attached to the cDNA(s). Subsequently, an adapter comprising a poly- or oligonucleotide sequence and, at its 3'-end, the second partner of the pair of molecules, is ligated via click reaction to the cDNA(s). Use of this adapter and a specific second primer allow for a highly effective amplification of the cDNA(s).

In the context of the present invention, mRNA refers to messenger RNA, i.e. a ribonucleic acid which is encoded by the DNA contained in a cell or organism and which can be translated into a protein by cellular mechanisms. Additionally, as defined here, the term mRNA can also encompass synthetic ribonucleic acids or mRNAs which include modified nucleotides. An mRNA is further meant to include nucleic acids which contain analogues of the sugar moiety. While natural mRNAs contain one or more bases selected from the group consisting of adenine, uracil, cytosine or guanine, non-native bases can also be included.

The method of the present invention can be applied to a sample which contains only one kind of mRNA. That means that the sample contains only mRNA having the same nucleotide sequence and the same number of nucleotides. In other embodiments of the invention, the sample can contain two or more different mRNAs. In the context of the present invention, the term "different mRNA" is intended to include not only mRNAs having different nucleotide sequences but also mRNAs which have the same nucleotide sequence but, due to whatever reasons, contain a different number of nucleotides.

In principle, the method of the invention can also be applied to other kinds of RNA, if amplification or sequencing of such RNA and especially of a mixture containing any kind of RNA is desired. The application of the inventive method to mRNA is preferred, and another preferred RNA to be included by such method is viral RNA in any form, i.e. also a viral RNA which is integrated into the genome of a host.

The inventive method includes a first step a), in which a first primer which is complementary to a sequence which is located at or near the 3'-end of the at least one mRNA, is added to the sample containing mRNA(s). Addition of this first primer to the sample occurs under conditions which allow for hybridization of the first primer to the complementary mRNA sequence.

The at least one first primer includes a sufficient number of nucleic acids which are complementary to the respective target mRNA to allow for an effective hybridization of the primer to the mRNA. The first primer can include any number of nucleotides which is considered useful in DNA/RNA amplification procedures. In a preferred embodiment, the first primer consists of at least 5 nucleotides, preferably at least 10 or at least 15 nucleotides. In such preferred embodiments, the first primer further consists of up to 60 nucleotides, preferably up to 50 nucleotides and most preferably of up to 45 nucleotides. Conditions to effect or favor hybridization of the at least one first primer to the at least one mRNA are well known to the skilled person and include those as described e.g. in J. Zhang et al., Biochem. J., 1999, 337, 231-241. Especially favorable conditions can also easily be determined by the skilled person by preliminary tests.

In a second step b) of the inventive method, starting from the 3'-end of the primer hybridized to the at least one mRNA, the mRNA is reverse-transcribed under conditions including addition of a reverse transcriptase and nucleotides to the sample to provide full-length cDNA(s) of the at least one mRNA. While the naturally occurring 4 types of nucleotides dATP, dCTP, dGTP and dTTP are usually employed in the context of the present invention, also artificial nucleotides can be included as long as they do not impair the cDNA formation of any further steps of the method of this invention. As for step a), conditions for the reverse transcription are also well known to the skilled persons.

In an intermediate step c), at least excess nucleotides used in step b) are removed. In preferred embodiments of the invention, also remaining mRNA(s), excess reverse transcriptase and excess first primer(s) can be removed during this step. In an especially preferred embodiment, a cDNA purification is performed according to methods known in the art and the purified cDNA included in the further steps of the method.

In the following step d), a dideoxy nucleotide (ddNTP), which is modified at the 3'-position to include a first partner of an azide/alkyne click-reaction pair, and a template-independent polymerase is added to the sample. The term "dideoxy nucleotide" or ddNTP stands for nucleotides which do not have 2'-nor 3'-hydroxyl groups. Within the context of the present invention, the ddNTPs include one of the bases adenine, thymine, cytosine or guanine. The modified dideoxy nucleotide is added to the cDNA sequence at its 3'-end by the polymerase and, since the dideoxy nucleotide does not contain the required 3'-hydroxyl group, no further chain extension can occur and the attached azide or alkyne group is available for a reaction of the 3'-end of the cDNA with a second click-reaction partner. In a preferred embodiment of the invention, the alkyne or azide group is attached to the 3'-position of the deoxyribose moiety of the ddNTP and also preferably, the ddNTP contains an azide group attached to the molecule.

In a further intermediate step e), at least an excess of the modified dideoxy nucleotide used in step d) is removed. In preferred embodiments of the invention, also during this step eventually remaining mRNA(s), excess reverse transcriptase and excess first primer(s) can be removed and/or the template-independent polymerase as used in step d).

In step f), an adapter molecule is added as the second click-reaction partner to the sample. The adapter molecule comprises an oligo- or polynucleotide sequence and contains attached to its 5'-end the second partner of the pair of azide and alkyne molecules. A click-reaction which occurs between the alkyne- and the azide groups ligates the adapter molecule to the 3'-end of the cDNA under formation of a triazole linkage. The adapter molecule serves for binding of a second primer in the next step of the inventive method via hybridization and subsequent amplification. Accordingly, the adapter molecule contains a number of nucleotides which is sufficient to allow for binding of such second primer, usually at least 6 nucleotides, preferably at least 10 and more preferably at least 15 nucleotides. In a preferred embodiment, the adapter molecule contains a maximum of 100 nucleotides, more preferable a maximum of 80 or 60 nucleotides. Furthermore, it is also considered a preferred embodiment of the invention to use an adapter molecule in step e, which contains an alkyne attached to its 5'-end.

The terms "click chemistry" or "click-reaction" or the like as used within the context of the present invention are intended to refer to all corresponding methods described in the art and the exemplary publications mentioned in the above "Background of the Invention" section. It is especially referred to WO 2019/063803 A1, the disclosure of which is specifically included for the purposes of the present invention, especially as far as it relates to conditions, reagents and methods to perform click reactions. Preferably, in the context of step f), a copper catalyzed azide-alkyne cycloaddition reaction (CuAAC) is performed and most preferably under conditions as described in WO 2019/063803 A1.

In the next step g), the above mentioned second primer is added which comprises a nucleotide sequence, which is complementary to all or part of the adapter molecule. The nucleotide sequence of the second primer is complementary to at least 6 nucleotides at the 5'-end of the nucleotide sequence of the adapter molecule and it contains at its 3'-end a nucleotide which is complementary to the dideoxy nucleotide at the 3'-end of the cDNA(s). Accordingly, the second primer hybridizes and binds to the ligated adapter/cDNA molecule at a position which overlaps the triazole linkage which was formed via click reaction.

In the further steps h) and i), the second primer is extended and one or more third primers are added which are identical to at least a part of the first primer(s), and the obtained cDNAs are amplified. The third primer can e.g. comprise less nucleotides than the first primer but still ensure hybridization. The third primer can also include a specific sequence which ensures hybridization and further sequences which are not complementary to the target sequence of the first primer on the at least one mRNA.

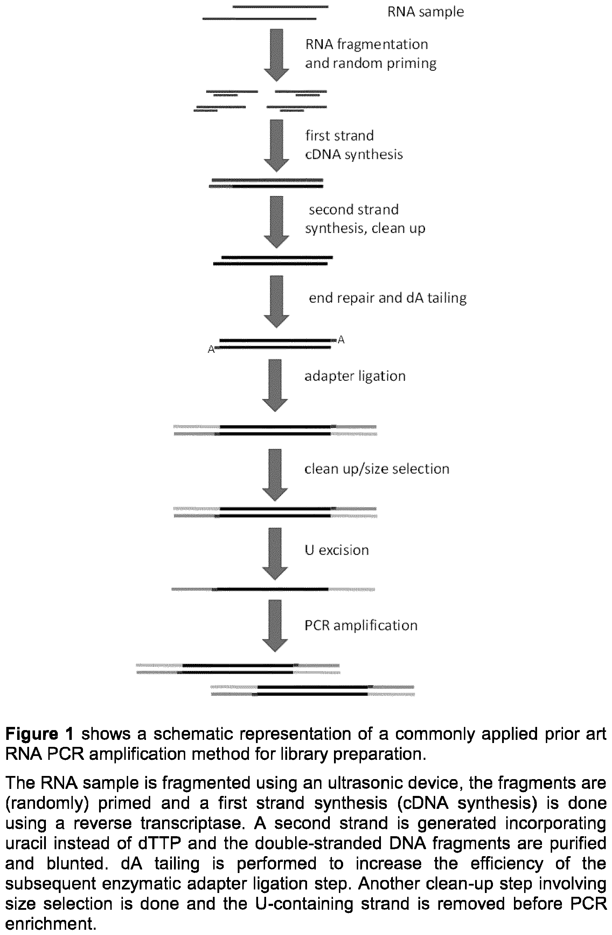

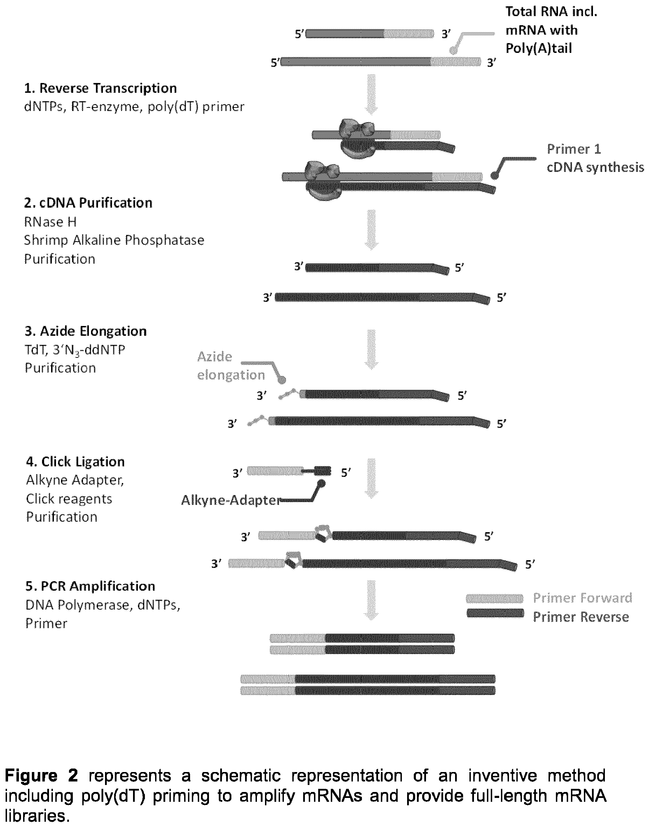

The method of the present invention for the first time allows to produce amplified cDNA for a specific RNA sample without the need for fragmentation of the mRNA and random priming as was required by commonly used processes, one of which is schematically represented in Fig. 1. Compared to such prior art amplification processes, the method of the present invention requires less process steps to arrive at a fully representative cDNA library for the complete mRNA or mRNAs of interest without the need for aligning sequence data for overlapping fragments. Also, the method of the invention produces full-length libraries from either random priming or specific priming. Additionally, the method of the present invention can be carried out with standard polymerases for library preparation workflows. A schematic representation of a method of the invention using poly(dT) priming is shown in Figs. 2.

As the first primers, various different alternatives and respective polynucleotides can be considered within the context of the invention. Firstly, random primers can be used as known from prior art methods. For this first alternative, rather short nucleotide sequences having a random sequence which hybridizes to random parts within the mRNA or mRNAs can be employed. The products of a process using such random first primers have different lengths.

In a second alternative and a preferred embodiment of the invention, specific primers are used instead of random primers. This alternative is especially applicable in cases where the sequence or identity of at least one mRNA contained in the sample is known. In cases where a sample is analyzed for the presence of one or more known mRNAs which could be present in the sample, corresponding specific primers can be used and allow for the detection of such known mRNAs.

In a further alternative which is also a preferred embodiment of the invention, a poly(dT) primer is used as the at least one first primer. A poly(dT) primer usually comprises 6 to 30 dT nucleotides. The primer is added to the sample under conditions which allow for hybridization to the poly(A) tail(s) of the at least one mRNA in the sample. All mRNA molecules contain a poly(A) tail at their 3'-end which consists of multiple adenosine monophosphates. The poly(A) tail is added to eukaryotic RNA via polyadenylation which takes place after the transcription of the gene is concluded. The poly(A) tail however does not have a coding function during translation. Thus, the poly(A) tail is an ideal target for primer hybridization and providing a corresponding poly(dT) primer does not require any knowledge about the sequence of the target mRNA. Furthermore, use of the poly(dT) primer allows for reverse transcription and amplification of the complete coding sequence of the mRNA(s) contained in a sample.

In certain embodiments, the invention also includes the use of different kinds of primers, i.e. random and specific primers or random and poly(dT) primer or specific and poly(dT) primers. This is especially useful in cases where primers also include a specific anchor or capture sequence. For instance, a specific primer can be included for detecting the presence of a known mRNA in a sample. The presence e.g. of a capture sequence attached to the 5'-end of such specific primer provides the option to, in a later step after amplification, attach the amplified cDNA obtained for this known mRNA to a solid support, to separate the cDNA attached to the solid support from other amplification products and to detect the presence of such cDNA. Other useful options provided by the inclusion of capture or anchor sequences attached to the first primer(s) are easily recognizable by the skilled person and are also encompassed within the context of the present invention.

In an especially preferred embodiment of the invention, a poly(dT) primer is used as the at least one first primer, which includes an anchor sequence. This anchor sequence provides an additional 5'-end-extension of usually 2 to 50 nucleotides. Preferably the anchor sequence contains at least 5 and more preferably at least 8 nucleotides. In further preferred embodiments, the anchor sequence contains a maximum of 40, more preferably a maximum of 30 nucleotides. This presence of such anchor sequence e.g. provides a possibility to ensure uniform positioning of the poly(dT) primer(s) used in the inventive method.

In a further preferred embodiment, 3'-alkyne- or 3'-azide-modified ddGTP or 3'-alkyne- or 3'-azide-modified ddCTP is added in step d) of the inventive method. It has been observed that the presence of one of the bases G or C at the 3'-end of the cDNA results in an especially efficient hybridization and attachment of the second primer in the following step g). The best binding results were obtained where the 3'-end of the cDNA is modified to include G by adding a 3'-alkyne- or 3'-azide modified ddGTP to the cDNA in step d), the most preferred modified dideoxy nucleotide being 3'-azido-2',3'-dideoxy GTP (AzddGTP).

In correspondence with the above mentioned preferred embodiments for step d), it is further preferred that the second primer in step g) contains at its 3'-end dG or dC, most preferably dC. The method of the present invention requires the second primer to contain at its 3'-end a nucleotide which is complementary to the nucleotide at the 3'-end of the cDNA(s). Accordingly, in the preferred embodiment, in which an alkyne- or azide-modified ddGTP is used in step d), the second primer contains dC at its 3'-end. In the other preferred embodiment, in which alkyne- or azide-modified ddCTP is included in step d), the second primer correspondingly contains dG at 3'-end. In line with the above disclosed most preferred embodiment using the modified ddGTP above, in such most preferred embodiment a second primer is used which contains dC at its 3'-end. In a further preferred embodiment of the present invention, the second primer includes a dC at the second position of its 3' end. It has been realized by the inventors that the presence of dC at this position where the second primer overlaps the triazole linkage formed by the click reaction, improves primer binding and subsequent primer extension considerably.

As the template independent polymerase in step d) preferably the enzyme terminal deoxynucleotidyl transferase (TdT) is used. The enzyme is a member of the X family of DNA polymerases and catalyzes the addition of nucleotides to the 3'-terminus of DNA molecules. Unlike most other DNA polymerases, TdT does not require a template. It can add nucleotides to a 3'-overhang as a preferred substrate, however, also works well in adding nucleotides to blunt or recessed 3'-ends. Accordingly, it has found important and wide spread application in molecular biology, i.e. in rapid amplification of cDNA ends (RACE), PCR and more recent enzymatic *de novo* gene synthesis.

In the context of the present invention, it is an option and also a preferred embodiment to include at least one of the primers and the adaptor molecules which include a moiety which comprises a detectable label or tag, or which allows for immobilization on a solid support. One of such options has already been described above for the first primer(s). Such moiety can for example be able to form a covalent bond to a solid support directly or indirectly via further molecules. For example in cases in which it is intended to capture amplified nucleic acids for further detection or sequencing, immobilization to a solid support can be highly advantageous and is commonly used in automated sequencing methods. In line with such embodiment, one of the primers or the adaptor used in the present invention can also be present already attached to a solid support and the method be performed in the presence of such solid support. As a solid support, any material can be included or added directly or at a later stage which enables capturing and also separation of the attached molecules from the reaction mixture. As solid support, a variety of materials and molecules can be included and used within the context of the present invention. E.g., flat or three-dimensional surfaces can be provided as a solid support, e.g., beads to which a primer or adaptor is attached or can be attached via subsequent binding reaction, can be used advantageously in this context. Furthermore, it is also possible to use one of the primers or the adaptor in a labelled form, i.e. including a detectable label or tag to allow for the detection of amplification products. Especially in cases, where specific primers are used, a corresponding detection of the presence of the amplified cDNAs is easy to be carried out by methods known to the skilled person.

Also, the above outlined options can be combined to provide for capture and detection of the amplified cDNAs. In an especially preferred further embodiment of the invention, at least one of the primers or adaptor used in the method of the invention is attached to a solid support or is connected or bound to a molecule which facilitates binding to a solid support, and at least one other of the primers and adaptor molecules contains a detectable label or a molecule via which a detectable label can be attached. These embodiments of the invention allow for a simple and preferably also automated detection of amplification products and, thus, the presence of a specific mRNA in the sample to be analyzed.

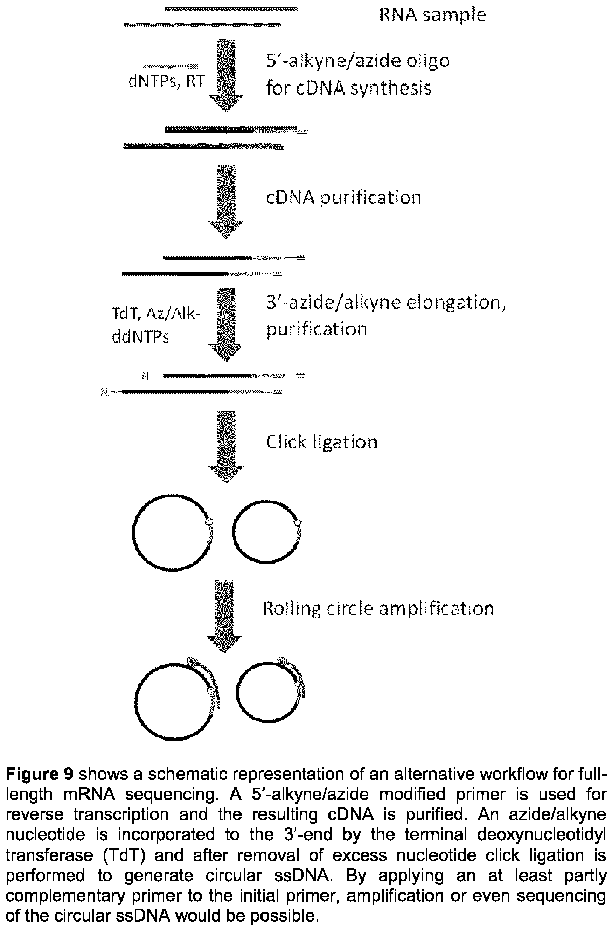

While the above described preferred examples cannot exhaustively list all possible variations and benefits of the inventive method, the skilled person can easily adapt the method to best suit the specific purpose of research or diagnosis. Especially combinations of the method of the invention with further known procedures are therefore included within the scope of the present invention.
A further alternative method of the invention is shown in Figure 9. In such alternative method, as the first primers, various different alternatives and respective polynucleotides can be considered within the context of the invention. Firstly,5'-azide/alkyne modified primers can be prepared by known solid-phase methods and used for reverse transcription as described above in steps a) and b). After purification in step c) and azide/alkyne modified ddNTP addition via a template-independent polymerase in step d) an azide and alkyne containing cDNA would be generated. After removal of excess nucleotide, click ligation could be performed to generate circular single stranded cDNAs under click conditions known to a PHOSITA (e.g. V. Cassinelli, et al. Angew. Chem. Int. Ed., 2015, 54, 7795-7798). In order to prevent concatenation, the double modified cDNAs are diluted before click ligation as described before (R. Kumar et al., J. Am. Chem. Soc., 2007, 129 (21), 6859-6864). Then, an alternative second primer which is complementary to at least a part of the first primer could be used instead of the second primer of step g) in order to amplify or sequence the template in a rolling circle amplification (BGI genomics NGS method). In this alternative method, accordingly, while most aspects described for the first inventive method still apply, addition of an adapter molecule described in step f) above, is not applicable or required anymore. The alternative second primer, however, would also contain at its 3'-end a nucleotide which is complementary to the dideoxy nucleotide at the 3' end of the cDNA (before circularization via click reaction).

In the context of the method of the invention and the above described alternative method, instead of performing an amplification via steps h) and i), it is also possible to directly perform sequencing according to known methods. E.g. a Sanger sequencing can be performed directly using the product obtained in step g) of the inventive method or the circular DNA/primer construct as shown at the bottom of Fig. 9 for the alternative method. Also single nucleotide sequencing methods can be applied using a product obtained by chain extension of the product of step g) pf the inventive method or of the circular DNA/primer construct of the alternative method.

A further subject of the present invention is the use of the inventive method for amplifying of one or more mRNAs contained in a sample for preparing a full length mRNA library. Especially in the context of the preferred embodiment of the invention, in which a poly(dT) primer is used as the at least one first primer, the method allows for amplification of the complete mRNA sequences contained in the sample. This is especially useful, when the sample contains the total mRNA of one or more types of cells of an individual or even the whole exome of an individual, that is the total mRNA which will be translated into protein in a specific cell or an organism.

A further subject of the present invention relates to the methods and use of the invention as detailed above and further including the determination of the sequence of the amplified mRNA or obtained mRNA library. Such method allows for the sequencing of the total mRNA of cells of an organism and even the whole exome of an organism. Providing such method is especially useful in medical research and clinical diagnostics, e.g., for variant mapping in complex disorders, investigation of genetic aberrations, determining the genetic basis for a disease or for diagnosing Mendilian disorders and other uses.

A further subject of the present invention provides a kit for performing at least one of the above-mentioned methods and uses of the invention. Such kit allows for amplifying at least one mRNA contained in the sample and comprises
a) a first primer which primer includes a sequence which is complementary to a sequence which is located at or near the 3'-end of the at least one mRNA to be amplified,
b) a reverse transcriptase,
c) a dideoxy nucleotide which is modified at the 3' position to include a first partner of a pair of azide and alkyne molecules,
d) a template-independent polymerase,
e) an adapter molecule comprising a polynucleotide sequence and attached to its 5'-end a second partner of said pair of azide and alkyne molecules
f) a second primer comprising a nucleotide sequence which is complementary to at least 6 of the nucleotides at the 5'-part of the adapter molecule and which contains at its 3'-end a nucleotide which is complementary to the dideoxy nucleotide of c),
g) a third primer which is identical to at least a part of the first primer. Furthermore, in preferred embodiments of the present invention, the kit can contain further useful reagents for performing the inventive methods, especially at least one of
h) an RNase,
i) all four kinds of naturally occurring nucleotides,
j) reagents for performing a click reaction,
k) buffers and solvents,
l) reagents for performing cDNA amplification
m) reagents for purification.

Kits for performing the alternative amplification method described above as well as a method in which a sequencing reaction is directly performed as outlined above are also included. The reagents contained in the kits for performing such methods can easily be adapted by the skilled person to include additional reagents or to set aside reagents which are not required for such alternative methods.

All information disclosed above with regard to one subject of the present invention is considered to equally apply in the context of other subjects for which this information, even if not explicitly repeated, has recognizable relevance within the context of the invention.

### Brief Description of the figures

**Figure 1** shows a schematic representation of a commonprior art PCR amplification of RNA for library preparation.
   The RNA sample is fragmented using an ultrasonic device, the fragments are (randomly) primed and a first strand synthesis (cDNA synthesis) is done using a reverse transcriptase. A second strand is generated incorporating uracil instead of dTTP and the double-stranded DNA fragments are purified and blunted. dA tailing is performed to increase the efficiency of the subsequent enzymatic adapter ligation step. Another clean-up step involving size selection is done and the U-containing strand is removed before PCR enrichment.
**Figure 2** represents a schematic representation of the inventive method including poly(dT) priming to amplify mRNAs and provide full-length mRNA libraries.

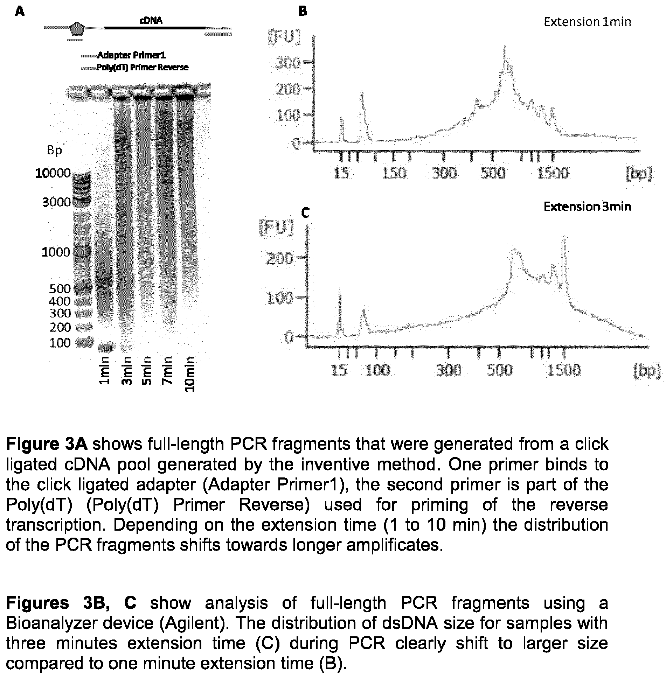

**Figure 3A** shows full-length PCR fragments that were generated from a click ligated cDNA pool generated by the inventive method. One primer binds to the click ligated adapter (Adapter Primer1), the second primer is part of the Poly(dT) (Poly(dT) Primer Reverse) used for priming of the reverse transcription. Depending on the extension time (1 to 10 min) the distribution of the PCR fragments shifts towards longer amplificates.
**Figures 3B****, C** show analysis of full-length PCR fragments using a Bioanalyzer device (Agilent). The distribution of dsDNA size for samples with three minutes extension time (C) during PCR clearly shift to larger size compared to one minute extension time (B).

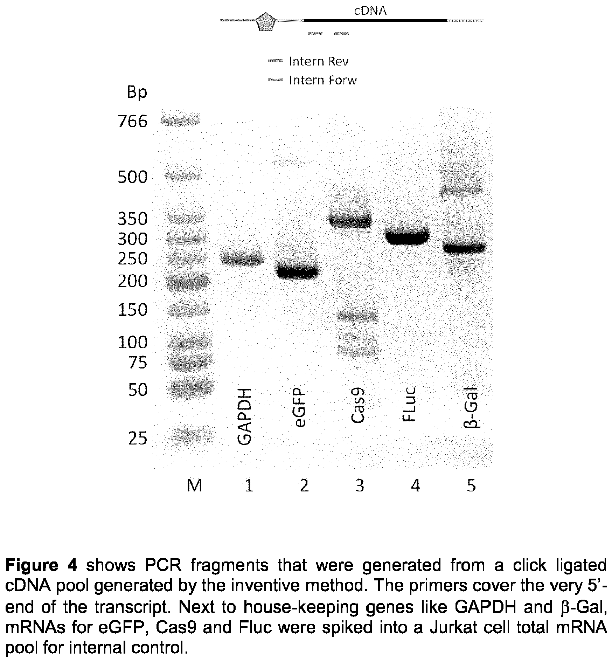

**Figure 4** shows PCR fragments that were generated from a click ligated cDNA pool generated by the inventive method. The primers cover the very 5'-end of the transcript. Next to house-keeping genes like GAPDH and β-Gal, mRNAs for eGFP, Cas9 and Fluc were spiked into a Jurkat cell total mRNA pool for internal control.

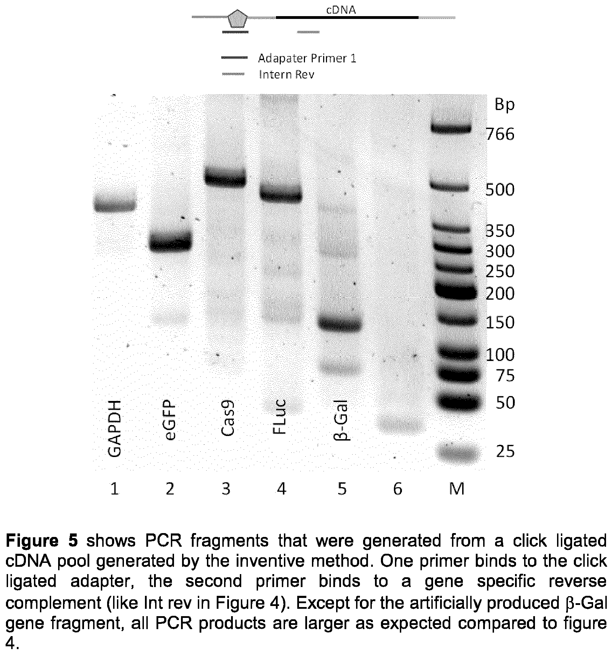

**Figure 5** shows PCR fragments that were generated from a click ligated cDNA pool generated by the inventive method. One primer binds to the click ligated adapter, the second primer binds to a gene specific reverse complement (like Int rev in Figure 4). Except for the artificially produced β-Gal gene fragment, all PCR products are larger as expected compared to figure 4.

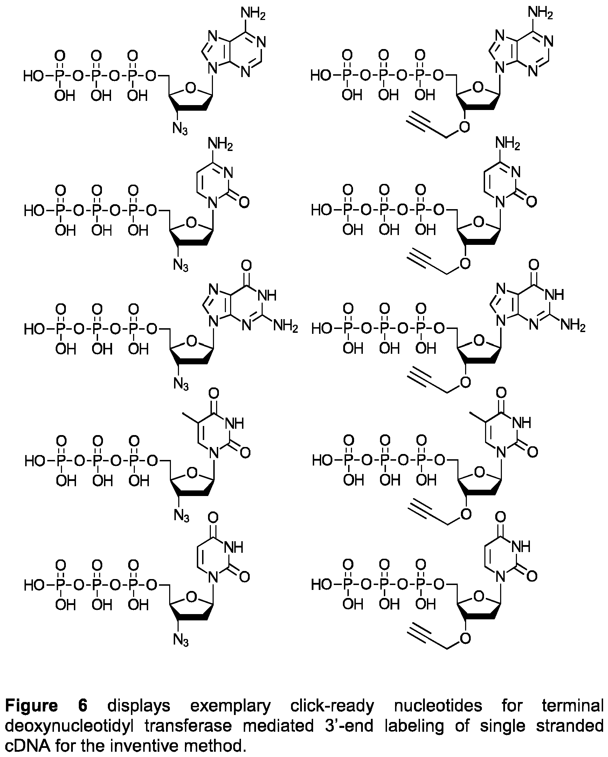

**Figure 6** displays exemplary click-ready nucleotides for terminal deoxynucleotidyl transferase mediated 3'-end labeling of single stranded cDNA for the inventive method.

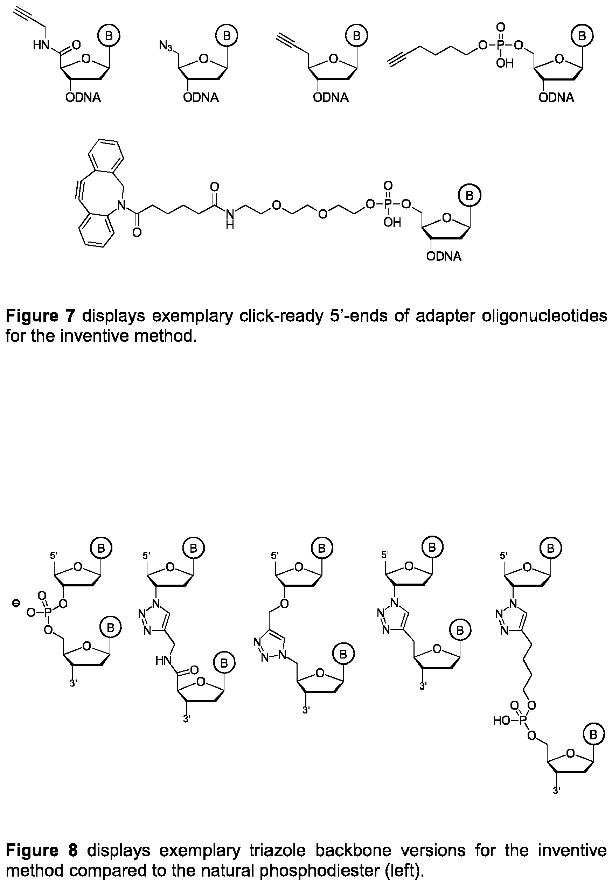

**Figure 7** displays exemplary click-ready 5'-ends of adapter oligonucleotides for the inventive method.
**Figure 8** displays exemplary triazole backbone versions for the inventive method compared to the natural phosphodiester (left).
**Figure 9** shows a schematic representation of an alternative workflow for full-length mRNA sequencing. A 5'-alkyne/azide modified primer is used for reverse transcription and the resulting cDNA is purified. An azide/alkyne nucleotide is incorporated to the 3'-end by the terminal deoxynucleotidyl transferase (TdT) and after removal of excess nucleotide click ligation is performed to generate circular ssDNA. By applying an at least partly complementary primer to the initial primer, amplification or even sequencing of the circular ssDNA would be possible.

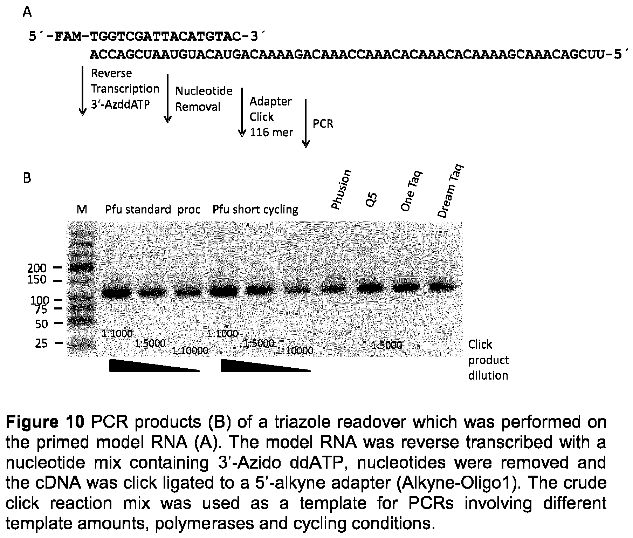

**Figure 10** PCR products (B) of a triazole readover which was performed on the primed model RNA (A). The model RNA was reverse transcribed with a nucleotide mix containing 3'-Azido ddATP, nucleotides were removed and the cDNA was click ligated to a 5'-alkyne adapter (Alkyne-Oligol). The crude click reaction mix was used as a template for PCRs involving different template amounts, polymerases and cycling conditions.

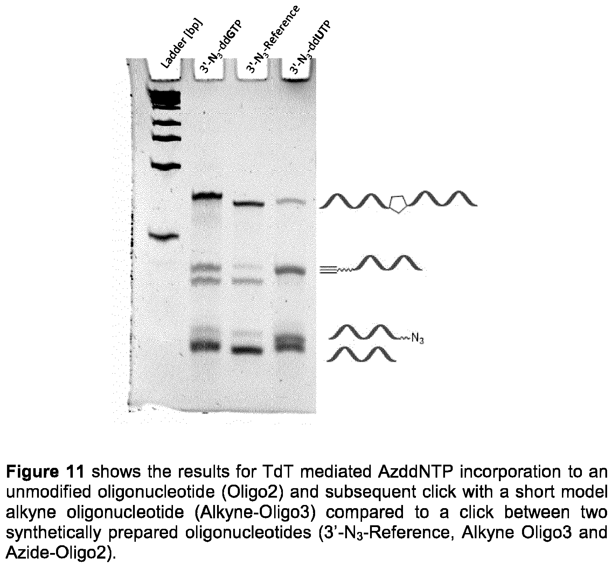

**Figure 11** shows the results for TdT mediated AzddNTP incorporation to an unmodified oligonucleotide (Oligo2) and subsequent click with a short model alkyne oligonucleotide (Alkyne-Oligo3) compared to a click between two synthetically prepared oligonucleotides (3'-N₃-Reference, Alkyne Oligo3 and Azide-Oligo2).

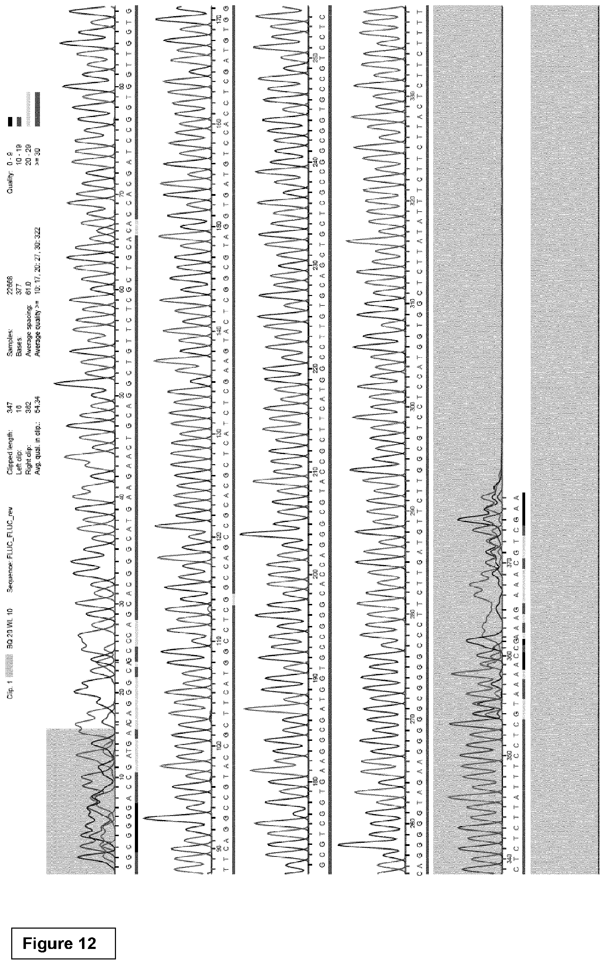

**Figure 12** shows the capillary electrophoresis trace of a Sanger sequencing, which was generated from a PCR fragment (Figure 5, Lane 4) treated according to the inventive method. The sequencing demonstrates that after the protocol the 5'-end of the mRNA remains intact. Here it was done for Fluc mRNA (firefly luciferase), which was spiked into the RNA pool before reverse transcription. The average quality value score (QV) is 54.34, i.e. > 99.999% certainty for the specific sequence.

The following Examples further illustrate the invention:

### Examples

### Example 1 (relating to Fig. 3)

For internal control, next to house-keeping genes like GAPDH, mRNAs (0.1 µg) of eGFP (Baseclick), CleanCap®Cas9 (Trilink), CleanCap®β-Gal (Trilink) and CleanCap®Fluc (Trilink) were spiked into 2µg of Jurkat cell total RNA pool. To this RNA pool, 1µL dNTP mix (10 mM) and 2 µL of the **Poly(dT) Primer** (100 µM) were combined to a total volume of 13 µL with RNase free H₂O. The mixture was incubated at 65°C for 5 min and cooled down to 0°C for 3 min to allow hybridization. For cDNA synthesis 4 µL 5x SuperScriptIV Buffer, 1 µL dithiothreitol (100 mM), 200 units of Superscript IV reverse transciptase were added and filled up with RNase free water to a total volume of 20 µL. The mixture was incubated at 50°C for 20 min, at 80°C for 10 min and cooled down to 4°C for 3 min. After cDNA synthesis 3 µL 10x RNase H Buffer, 1 µL RNase A (10 mg/mL), 1.4 µL RNase H (5 U/µL) and 4 µL shrimp alkaline phosphatase (1 U/µL) and 0.6 µL dH₂O were added to remove RNA and excess of nucleotides. The mixture was incubated at 37 C for 25 min, at 65 C for 15 min and cooled down to 0 C for 3 min and then cleaned by a spin column method (BaseClean Kit) according to manufacturers' instruction for PCR products and eluted with 17 µL dH₂O.

The purified mixture was directly used for **azide elongation** with 3'-N₃-ddGTP. To 17 µL purified cDNA mixture 5 µL 5x TdT Buffer, 1 µL 3'-N₃-ddGTP (10 mM) and 2 µL of terminal deoxynucleotidyl transferase (20 U/µL) was added. The solution was incubated at 37°C for 1 h and cooled down to 0 C for 3 min. Modified cDNA was purified by a spin column method (BaseClean Kit) according to manufacturers' instruction for PCR products and eluted with 9 µL.

**Click ligation** was performed using 9 µL of the purified and azide-modified cDNA, 0.5 µL of **Alkyne Adapter** (100 µM), 2.5 µL Activator²₄₀ (baseclick GmbH), 0.5 µL dH₂O and two reactor pellets. The reaction mixture was incubated at 45 C, 600 rpm, for 40 min. After the reaction, the supernatant was transfered to a new vial. The pellets were washed with 12.5 µL water and the supernatant was transferred to the supernatant before. The click ligated cDNA was cleaned using a spin column method (BaseClean Kit) according to manufacturers' instruction for PCR products and eluted with 10 µL.

After click ligation, the cDNA pool was amplified using untargeted primer **(Adapter Primer1** and **Poly(dT) Primer Reverse)** to obtain a full-length mRNA library. In a 200 µL reaction vial 11.5 µL dH₂O, 4 µL 5x OneTaq Buffer, 1 µL **Adapter Primer1** (10 µM), 1 µL **Poly(dT) Primer Reverse** (10 µM), 0.4 µL dNTP Mix (10 mM), 2 µL of the purified click ligation mixture and 0.13 µL OneTaq DNA Polymerase (5000 U/µL) (New England Biolabs) were combined. The sample was subjected to a thermal cycling program in a thermocycler (BIORAD).

As a standard cycling following conditions were used with different extension times (step 4):

| **step** | **temperature** | **duration** | |
|---|---|---|---|
| 1 | 94 C | 2 min | |
| 2 | 94 C | 20 s | |
| 3 | 54 C | 20 s | 20 x |
| 4 | 68 C | 1min/3min/5min/7min/10min | |
| 5 | 68 C | 5 min | |

The PCR mixture was cleaned using a spin column method (BaseClean Kit) according to manufacturers' instruction for PCR products and eluted with 10 µL dH₂O. In **Figure 3A** 3 µL aliquots of each PCR reaction were analyzed on 1.5% agarose gels (10 x 15 cm) prepared in TAE buffer (20 mM TRIS, 10 mM acetic acid, 0.5 mM EDTA).

For bioanalyzer measurements as shown in Figure 3B and C, 1 µL purified aliquots of generated full-length PCR fragments from an mRNA pool (Figure 3A Lane 1 extension time 1 minute and lane 2 extension time 3 minutes) were measured using the DNA 1000 Kit (Agilent) and a bioanalyzer device (Agilent 2100).

**Oligonucleotides:**

| **Name** | **Sequence** | **Modification** |
|---|---|---|
| Poly(dT) Primer | GTG ACT GGA GTT CAG ACG TGT TTT TTT TTT TTT TTT TTT TVN | V = dA, dC, dG; N = dA, dC, dG, dT |
| Alkyne Adapter | XTAGATCGGAAGAGCGTCGTGT AGGGAAAGAGTGTAGATCTCGG TGGTCGCCGTATCATT | X = 5'-Hexynyl linker |
| Adapter Primer1 | CGA CGC TCT TCC GAT CTA C | No |
| Poly(dT) Primer Reverse | GTG ACT GGA GTT CAG ACG TGT | No |

### Modifications:

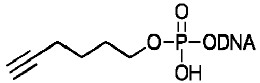

### Example 2 (relating to Fig. 4)

After click ligation (procedure described in example 3), specific genes were amplified from the cDNA pool using targeted primer (various Intern Primer Reverse and Forward, see table below), which result in specific PCR fragments. In a 200 µL reaction vial 11.5 µL dH₂O, 4 µL 5x OneTaq standard reaction buffer, 1 µL **Intern Reverse Primer** (10 µM), 1 µL **Intern Forward Primer** (10 µM), 0.4 µL dNTP mix (10 mM), 2 µL of the purified and click ligated cDNA mixture and 0.13 µL OneTaq DNA Polymerase (5000 U/µL) (New England Biolabs) were combined. The sample was subjected to a thermal cycling program in a thermocycler (BIORAD).

| **step** | **temperature** | **duration** | |
|---|---|---|---|
| 1 | 94 C | 2 min | |
| 2 | 94 C | 20 s | |
| 3 | 54 C | 20 s | 20 x |
| 4 | 68 C | 1 min | |
| 5 | 68 C | 5 min | |

5 µL unpurified aliquots of each PCR amplification were analyzed on 1.5% agarose gels (10 x 15 cm) prepared in TAE buffer (20 mM TRIS, 10 mM acetic acid, 0.5 mM EDTA).

### Oligonucleotides:

| **Name** | **Sequence** | **Modification** |
|---|---|---|
| GAPDH Intern Reverse Primer | GGA GGG ATC TCG CTC CTG | No |
| GAPDH Intern Forward Primer | ATG GGG AAG GTG AAG GTC GG | No |
| eGFP Intern Reverse Primer | GCCGTAGGTCAGGGTGGTC | No |
| eGFP Intern Forward Primer | GCGAACTAGTAAGCAAGGAGG | No |
| Cas9 Intern Reverse Primer | CCA CCA GGA AGC TCT CCT CC | No |
| Cas9 Intern Forward Primer | ATGGCCCCCAAGAAGAAGCG | No |
| β-Gal Intern Reverse Primer | GCA CTC CAG CCA GCT CTC | No |
| β-Gal Intern Forward Primer | ATGAGCTTCACCCTGACCAACA | No |
| FLUC Intern Reverse Primer | GTT GTA GAT GTC GTT GGC GGG | No |
| FLUC Intern Forward Primer | ATG GAG GAC GCC AAG AAC ATC A | No |

### Example 3 (relating to Fig. 5 and Fig. 12)

After click ligation (procedure described in example in Figure 3), specific genes were amplified from the cDNA pool using a gene specific primer and a common adapter primer targeted primer (various Intern Primer Reverse and Adapter Primer 1, see table below), which result in specific PCR fragments. In a 200 µL reaction vial 11.5 µL dH₂O, 4 µL 5x OneTaq standard reaction buffer, 1 µL **Intern Reverse Primer** (10 µM), 1 µL **Adapter Primer1** (10 µM), 0.4 µL dNTP mix (10 mM), 2 µL of the purified and click ligated cDNA mixture and 0.13 µL OneTaq DNA Polymerase (5000 U/µL) (New England Biolabs) were combined. The sample was subjected to a thermal cycling program in a thermocycler (BIORAD).

| **step** | **temperature** | **duration** | |
|---|---|---|---|
| 1 | 94 C | 2 min | |
| 2 | 94 C | 20 s | |
| 3 | 54 C | 20 s | 20 x |
| 4 | 68 C | 1 min | |
| 5 | 68°C | 5 min | |

5µL unpurified aliquots of each PCR amplification were analyzed on 1.5% agarose gels (10 x 15 cm) prepared in TAE buffer (20 mM TRIS, 10 mM acetic acid, 0.5 mM EDTA).
For Sanger sequencing as shown in **Figure 12****,** the generated PCR fragment of Fluc (firefly luciferase) was cleaned using a spin column method (BaseClean Kit) according to manufacturers' instruction for PCR products and eluted with 20 µL dH₂O. This template and the specific primer (FLUC Intern Reverse Primer) were prepared according to and send to Eurofins Genomics (Ebersberg, Germany) for Sanger sequencing.

**Oligonucleotides:**

| **Name** | **Sequence** | **Modification** |
|---|---|---|
| Adapter Primer1 | CGA CGC TCT TCC GAT CTA C | No |
| GAPDH Intern Reverse Primer | GGA GGG ATC TCG CTC CTG | No |
| eGFP Intern Reverse Primer | GCCGTAGGTCAGGGTGGTC | No |
| Cas9 Intern Reverse Primer | CCA CCA GGA AGC TCT CCT CC | No |
| β-Gal Intern Reverse Primer | GCA CTC CAG CCA GCT CTC | No |
| FLUC Intern Reverse Primer | GTT GTA GAT GTC GTT GGC GGG | No |

### Example 4 (relating to Fig. 10)

The feasibility of the triazole readover was exemplified for reverse transcription of a **Model RNA** sequence. The RNA was hybridized to **Primer1** and then reverse transcribed in the presence of 200 µM dTTP, dGTP, dCTP and 3'-azido-ddATP using MuLV reverse transcriptase. Nucleotides and enzyme were removed by purification of the cDNA using the nucleotide removal kit (QIAGEN) according to manufacturers' instructions. **Alkyne Oligo1** was clicked to the purified cDNA in a 200 µL reaction vial with a single reactor pellet (600-800 µm, containing elemental copper) in a total 12.5 µL reaction mix and incubated at 45 C for 60 min. The reaction mix consisted of 800 µM THPTA, 20 mM MgCl₂, 5% DMSO, 7 µM of **Alkyne Oligo1** and about 4 µM purified cDNA. dH₂O was used to adjust the volume to a final 12.5 µL if necessary.

After the incubation the sample was briefly spinned down and the supernatant was transferred to a new vial to stop the reaction. The crude click reaction was diluted 1:1000, 1:5000 and 1:10000 (max. 4 nM, 0.8 nM and 0.4 nM) for PCR amplification without further purification.

In a 200 µL reaction vial, PCR amplifications were prepared in a total volume of 20 µL. Click reaction dilutions were combined with 200 µM dNTPs, 10 pmol of **Primer2** and **Primer3** and 1 U polymerase. For the various polymerases, Pfu, Phusion, Q5, One Taq and Dream Taq buffers were used according to manufacturers' recommendations. The samples were subjected to a thermal cycling program in a thermocycler (BIORAD).

As a standard cycling condition following conditions were used:

| **step** | **temperature** | **duration** | |
|---|---|---|---|
| 1 | 95 C | 2 min | |
| 2 | 95 C | 15 s | |
| 3 | 51 C | 20 s | 25 x |
| 4 | 72 C | 30 s | |
| 5 | 72 C | 2 min | |

For the Pfu polymerase different template dilutions and an alternative cycling condition were studied:

| **step** | **temperature** | **duration** | |
|---|---|---|---|
| 1 | 95 C | 2 min | |
| 2 | 95 C | 15 s | |
| 3 | 52 C | 5 s | 25 x |
| 4 | 72 C | 20 s | |
| 5 | 72 C | 2 min | |

After the incubation the sample was briefly spinned down and an aliquot was analyzed on 3% agarose gels (10 x 15 cm) prepared in TAE buffer (20 mM TRIS, 10 mM acetic acid, 0.5 mM EDTA).

Samples were prepared with 20% purple loading dye (NEB), and low molecular weight DNA ladder (25-766 bp, NEB, N3233) was prepared accordingly; usually 0.5 µL marker were used in 5 µL loading volume. Gels were run in TAE buffer applying constant power (10 W, max. 500 V, max. 100 mA) for 60 min. Then, gels were incubated in a freshly prepared 1:10000 ethidium bromide dilution for 15 min and then destained in dH₂O for 15 min. For visualization a Gel Doc EZ Imager (BIORAD) was used.

**Oligonucleotides:**

| **Name** | **Sequence** | **Modification** |
|---|---|---|
| Alkyne Oligo1 | XAA TGA TAC GGC GAC CAC CGA GAT CTA CAC TCT TTC CCT ACA CGA CGC TCT TCC GAT CT | X = 5'-alkyne dT |
| Model RNA | UUC GAC AAA CGA AAA CAC AAA CAC AAA CCA AAC AGA AAA CAG UAC AUG UAA UCG ACC A | n.r. |
| Primer1 | FAM-TGG TCG ATT ACA TGT AC | FAM = fluorescein |
| Primer2 | TGG TCG ATT ACA TGT ACT GTT TT | n.r. |
| Primer3 | AGA TCG GAA GAG CGT CG | n.r. |

Resulting cDNA after reverse transcription:

5'-FAM-TGG TCG ATT ACA TGT ACT GTT TTC TGT TTG GTT TGT GTT
TGT GTT TTC GTT TGT CGA-N3

Resulting click product: 
5'-FAM-TGG TCG ATT ACA TGT ACT GTT TTC TGT TTG GTT TGT GTT
TGT GTT TTC GTT TGT CGA TAA TGA TAC GGC GAC CAC CGA GAT
CTA CAC TCT TTC CCT ACA CGA CGC TCT TCC GAT CT-3'
 **AT** = A and T joined *via* backbone mimic

Resulting PCR product:

5'-TGG TCG ATT ACA TGT ACT GTT TTC TGT TTG GTT TGT GTT TGT
GTT TTC GTT TGT CGA TAA TGA TAC GGC GAC CAC CGA GAT CTA
CAC TCT TTC CCT ACA CGA CGC TCT TCC GAT CT-3'

### Modifications:

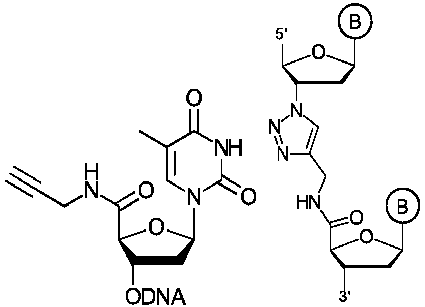

### Example 5 (relating to Fig. 11)

In a final volume of 25 µL the samples contained 1 µM **Oligo2,** 1 mM 3'-Azido-2',3'-dideoxyguanosine-5'-triphosphate or 3'-Azido-2',3'-dideoxyuridine and TdT-enzyme (2 U/µl) in 1x terminal nucleotidyl transferase buffer (25 mM Tris-HCI (pH 7.2), 200 mM potassium cacodylate, 0.01% (v/v) Triton X-100, 1 mM CoCl₂) for TdT-reactions. The reactions were incubated overnight (12-15 h) at 37 C and stopped by heating to 70 C for 10 min. The mixture was purified using the QIAquick Nucleotide Removal Kit and eluted in 30 µL water.

In a 200 µL reaction vial two reactor pellets (600-800 µm, containing elemental copper) were combined with 12.5 µL reaction mix and incubated at 45 C for 60 min. The click reaction mixture consisted of 800 µM THPTA, 40 mM MgCl₂, 5% (v/v) DMSO in H₂O, 1 µM of **Alkyne-Oligo3** and about 0.3 µM purified azide oligonucleotide from TdT reaction. As a reference a reaction mixture consisted of 800 µM THPTA, 40 mM MgCl₂, 5% (v/v) DMSO in H₂O, 1 µM of **Alkyne-Oligo3** and about 1 µM of **Azide-Oligo2** was mixed.

After the incubation the sample was briefly spinned down and the supernatant was transferred to a new vial to stop the reaction. Samples were analyzed on a 20% polyacrylamide gel. As a reference low molecular weight DNA ladder (25-766 bp, NEB, N3233) was used.

For denaturing PAGE of oligonucleotides, samples were mixed with urea (50 v/v) and loaded on 20% denaturing polyacrylamide gels (7.5mL Rotiphorese® Sequencing gel concentrate 750 µL Rotiphorese® Sequencing buffer concentrate, 2.09 mL bidistilled water, 3 mL 6 M urea solution, 1.5 mL 10 x Tris-borate-EDTA (TBE) buffer (1 M tris, 1 M H₃BO₃, 25 mM EDTA), 150 µL ammonium persulfate (10% (w/v), 10 µL tetramethyl ethylene diamine (W x D x H = 7.5 x 0.1 x 8.3 cm). Gel electrophoreses were performed in 0.5 x TBE buffer at 150 V for 2 h. The gel was stained in ethidium bromide solution and washed with water. Afterwards the bands were visualized using Gel Doc™ EZ System by BioRad and analyzed using Image Lab™ software.

**Oligonucleotides:**

| **Name** | **Sequence** | **Modification** |
|---|---|---|
| Oligo2 | TTG GTA TCG CTA TCG CTA TGG | n.r. |
| Alkyne-Oligo3 | XAA AAA AAC CAT GAA CAA AAT GTG ACT CAT ATC | X = 5'-Hexynyl linker |
| Azide-Oligo2 | TTG GTA TCG CTA TCG CTA TGG Z | Z= 3'-Azide, 5-methylcytosine |

### Modifications:

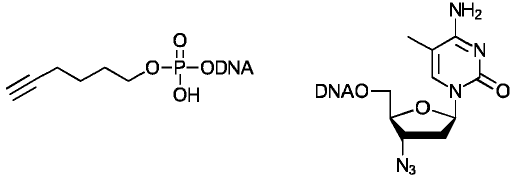

## Claims

1. Method for amplifying at least one RNA, preferably mRNA contained in a sample, the method comprising:
a) providing at least one first primer, which first primer includes a sequence which is complementary to a sequence which is located at or near the 3'-end of the at least one mRNA to be amplified, and adding said at least one first primer to the sample under conditions which allow for hybridization of at least one first primer to at least one mRNA,
b) reverse transcribing the at least one mRNA under conditions including addition of a reverse transcriptase and nucleotides to the sample to provide full length cDNA(s) of the at least one mRNA,
c) purifying the sample at least from excess nucleotides
d) adding a dideoxy nucleotide, which dideoxy nucleotide is modified at the 3'-position to include a first partner of a pair of azide and alkyne molecules, and a template-independent polymerase to attach a single 3'-azide- or 3'-alkyne modified dideoxy nucleotide at the 3'-end of the cDNAs obtained in step b),
e) purifying the sample at least from excess modified dideoxy nucleotide added in step d),
f) adding an adapter molecule, the adapter molecule comprising a polynucleotide sequence and, attached to its 5' end, a second partner of said pair of azide and alkyne molecules, under conditions to perform a click reaction and to ligate the adapter molecule to the 3'-modified cDNAs obtained in step d) under formation of a triazole linkage,
g) adding a second primer, the second primer comprising a nucleotide sequence which is complementary to at least 6 of the nucleotides at the 5'-end of the adapter molecule and contains at its 3'-end a nucleotide which is complementary to the dideoxy nucleotide at the 3'-end of the cDNAs, to effect hybridization and binding of the second primer to the ligated adapter/cDNA molecule obtained in step e) at a position overlapping the triazole linkage,
h) adding a third primer which is identical to at least a part of the first primer, and
i) amplifying the full length cDNAs.

2. Method according to claim 1, wherein in step a) as the at least one first primer a poly(dT) primer, preferably including a 5'-extension of 2 to 50 nucleotides as anchor sequence, is provided and added to the sample under conditions which allow for hybridization to the poly(A) tail(s) of the at least one mRNA present in the sample.

3. Method according to claim 1 or 2, wherein in step d) 3'-alkyne- or 3'-azide-modified ddGTP or 3'-alkyne- or 3'-azide-modified ddCTP, preferably 3'-azide-modified ddGTP, most preferably 3'-azido-2',3'-dideoxy GTP (AzddGTP) is used as the dideoxy nucleotide.

4. Method according to claims 1, 2 or 3, wherein in step g) a second primer is added which at the 3'-end contains dC or dG, preferably dC.

5. Method according to anyone of claims 1 to 4, wherein in step g) a second primer is added which at the second position on the 3'-end of the nucleotide sequence contains dC or dG, preferably dC .

6. Method according to anyone of claims 1 to 5, wherein in step d) terminal deoxynucleotidyl transferase (TdT) is used as the template-independent polymerase.

7. Method according to anyone of claims 1 to 6, wherein in step f) an alkyne is preferably attached to the 5'-end of the adapter molecule.

8. Method according to anyone of claims 1 to 7, wherein in step f) the click reaction comprises a copper catalyzed azide-alkyne cycloaddition (CuAAC).

9. Use of a method according to anyone of claims 1 to 8 for preparing a full length mRNA library from a sample containing a plurality of mRNA molecules.

10. Use according to claim 9, wherein the sample containing a plurality of mRNA molecules comprises the total mRNA of one or more types of cells or the whole exome of an individual.

11. Method for sequencing a plurality of mRNAs contained in a sample, the total mRNA of one or more types of cells or the whole exome of an individual, the method comprising providing a sample containing such plurality of mRNAs, such total cell mRNA or exome of an individual, preparing a library of full-length mRNA by performing a method according to anyone of claims 1 to 8 or in accordance with the use of claim 9, and determining the sequence of the amplified mRNA or obtained mRNA library.

12. Method according to claim 11, wherein long-read sequencing technology is applied.

13. Method according to claim 11 or 12 for variant mapping in complex disorders, investigation of genetic aberrations.

14. Kit for amplifying at least one mRNA contained in a sample, the kit comprising:
a) a first primer which primer includes a sequence which is complementary to a sequence which is located at or near the 3'-end of the at least one mRNA to be amplified,
b) a reverse transcriptase,
c) a dideoxy nucleotide which is modified at the 3' position to include a first partner of a pair of azide and alkyne molecules,
d) a template-independent polymerase,
e) an adapter molecule comprising a polynucleotide sequence and attached to its 5'-end a second partner of said pair of azide and alkyne molecules
f) a second primer comprising a nucleotide sequence which is complementary to at least 6 of the nucleotides at the 5'-part of the adapter molecule and which contains at its 3'-end a nucleotide which is complementary to the dideoxy nucleotide of c),
g) a third primer which is identical to at least a part of the first primer.

15. Kit according to claim 14, which further comprises at least one of:
h) an RNase,
i) all four kinds of naturally occurring nucleotides,
j) reagents for performing a click reaction,
k) buffers and solvents,
I) reagents for performing cDNA amplification
m) reagents for purification.
